# EUROPÄISCHE PATENTANMELDUNG

(11) **EP 4 197 584 A1**
(43) Veröffentlichungstag der Anmeldung: **21.06.2023**
(21) Anmeldenummer: 23156074.9
(22) Anmeldetag: 04.11.2014
(51) Int. Cl.: A61M 39/10, F16L 37/248, F16L 37/38

(54) **KONNEKTOR MIT CODIERELEMENTEN**

(30) Priorität: 06.11.2013 DE 102013018639
(62) Teilanmeldung aus: 18163169.8
(71) Anmelder: Fresenius Medical Care Deutschland GmbH, 61352 Bad Homburg (DE)
(72) Erfinder: Weber, Tobias, 66606 St. Wendel (DE); Berlich, Robert, 66606 St. Wendel (DE); Mager, Gerhard, 61352 Bad Homburg (DE)
(74) Vertreter: Herrmann, Uwe

(57) **Zusammenfassung**

Die Erfindung betrifft einen Konnektor umfassend wenigstens ein erstes Konnektorteil und wenigstens ein zweites Konnektorteil, wobei die Konnektorteile miteinander fluiddicht verbindbar und über wenigstens ein Verschlusselement aneinander arretierbar sind, wobei eines der Konnektorteile wenigstens ein im geschlossenen Zustand durchflusssperrendes, und öffenbares Dichtelement, insbesondere ein Septum oder eine Dichtscheibe, und das andere der Konnektorteile wenigstens ein Öffnungselement zum Öffnen des Dichtelementes aufweist und wobei eines der Konnektorteile wenigstens einen Vorsprung und das andere der Konnektorteile wenigstens eine Aufnahme aufweist, wobei das Öffnungselement und das Dichtelement derart zueinander angeordnet sind, dass das Öffnungselement das Dichtelement nur dann öffnet, wenn der Vorsprung in die Aufnahme eingeführt ist.

## Beschreibung

Die vorliegende Erfindung betrifft einen Konnektor umfassend wenigstens ein erstes Konnektorteil und wenigstens ein zweites Konnektorteil, wobei die Konnektorteile miteinander fluiddicht verbindbar und über wenigstens ein Verschlusselement aneinander arretierbar sind, wobei eines der Konnektorteile wenigstens ein im geschlossenen Zustand durchflusssperrendes, und vorzugsweise dichtend öffenbares Dichtelement, insbesondere ein Septum oder eine Dichtscheibe, und das andere der Konnektorteile wenigstens ein Öffnungselement zum Öffnen des Dichtelementes aufweist und wobei eines der Konnektorteile wenigstens einen Vorsprung und das andere der Konnektorteile wenigstens eine Aufnahme aufweist.

In der extrakorporalen Blutbehandlung werden üblicherweise Behandlungsflüssigkeiten in Beuteln bereitgestellt. Je nach Therapieart und Patientenbedürfnissen wird dabei eine Vielzahl von verschiedenen Behandlungsflüssigkeiten bereitgestellt, die sich in Zusammensetzung und Konzentration der einzelnen Lösungskomponenten unterscheiden können.

Die Beutel verfügen im Allgemeinen über ein Konnektorteil, das mit einem komplementären Konnektorteil, d.h. mit einem Konnektorteil, mit dem das Beutelkonnektorteil verbindbar ist verbunden werden kann, um die Behandlungsflüssigkeit für die Behandlung aus dem Beutel zu entnehmen und beispielsweise in ein Schlauchsystem zu überführen, an dem das komplementäre Konnektorteil angeordnet ist.

Insbesondere in dem Fall, dass mehrere Behandlungsflüssigkeiten in der Therapie gleichzeitig verwendet werden, besteht die Gefahr, dass einzelne Beutel durch Verwechslung nicht mit den dafür vorgesehenen Anschlüssen eines Schlauchsystems verbunden werden. Dies kann dazu führen, dass die falsche Flüssigkeit für die Blutbehandlung oder eine sonstige Behandlung verwendet wird und im schlimmsten Fall einem Patienten infundiert wird, was mit erheblichen gesundheitlichen Risiken einhergehen kann.

Daher besteht das Bedürfnis, einen Konnektor bereitzustellen, der es ermöglicht, dass ein Beutel eine Art von Behandlungsflüssigkeit auch nur genau einem dafür vorgesehenen Anschluss zugeordnet werden kann, sodass Verwechslungen ausgeschlossen sind. Aus dem Stand der Technik gemäß der WO 09/024807 A1 ist es bekannt, einen Konnektor aus zwei Konnektorteilen bereitzustellen, bei dem an einem Konnektorteil Arretierungsvorsprünge und an dem anderen Konnektorteil komplementäre Vorsprünge angeordnet sind, die im konnektierten Zustand zusammenwirken und auf diese Weise ein Konnektierungssignal "abgeben". Des Weiteren ist es aus der WO1 1/131783 A2 bekannt, ein Medikamentenbehältnis an eine Injektionsvorrichtung über einen Konnektor zu verbinden. Darin ist offenbart, die Konnektierung von komplementären Konnektorteilen zu codieren. Verwendet wird ein Codiervorsprung, der in eine Nut eingreift.

Der vorliegenden Erfindung liegt die Aufgabe zugrunde, einen Konnektor der eingangs genannten Art dahingehend weiterzubilden, dass besonders zuverlässig verhindert werden kann, dass einem Patienten aufgrund eines Versehens eine falsche Behandlungsflüssigkeit zugeführt wird.

Diese Aufgabe wird durch einen Konnektor mit den Merkmalen des Anspruchs 1 gelöst. Danach ist vorgesehen, dass das Öffnungselement und das durchflusssperrrende und aufmachbare, d.h. öffenbare Dichtelement, insbesondere das Septum, eine Dichtscheibe oder dergleichen derart zueinander angeordnet sind, dass das Öffnungselement das Dichtelement, insbesondere das Septum etc. nur dann öffnet, wenn der Vorsprung in die Aufnahme eingeführt ist.

Vorzugsweise handelt es sich bei den beiden Konnektorteilen um Konnektorteile, die nach Art eines Bajonett-Verschlusses miteinander verbindbar sind.

Vorzugsweise befinden sich die Aufnahme und der Vorsprung umfänglich an den Konnektorteilen.

Darüber hinaus ist es denkbar, dass ein Konnektorteil ein Öffnungselement, wie beispielsweise einen Konus aufweist, der in einer bestimmten Position der Konnektorteile zueinander mit einem Septum oder dergleichen, beispielsweise mit einer geschlitzten Dichtung so zusammenwirkt, dass das Öffnungselement das Septum öffnet. Dies ist jedoch nur möglich, wenn der Vorsprung in der Aufnahme aufgenommen wird, was wiederum nur dann der Fall ist, wenn die beiden Konnektorteile zueinander passen, d.h. komplementär sind, und was nicht der Fall ist, wenn die Konnektorteile nicht zueinander passen. Auf diese Weise wird verhindert, dass bei nicht zueinander komplementären Konnektorteilen ein Öffnen des Septums erfolgt, da in diesem Fall die Vorsprünge nicht oder jedenfalls nicht so in die Aufnahme eingeführt werden können, dass das Öffnungselement das Septum öffnet.

Dies bedeutet, dass das Septum oder dergleichen in diesem Fall geschlossen bleibt und die Verabreichung einer falschen Flüssigkeit zum Patienten ausgeschlossen ist.

Der Vorsprung und die Aufnahme, die als Codierelemente dienen haben somit nicht nur die Aufgabe, die beiden Konnektorteile z. B. formschlüssig aneinander zu halten bzw. dienen nicht nur als Codierelemente, sondern liefern auch eine Lösung für das Sicherheitsbedürfnis, wonach bei einer Fehlkonnektion, d.h. bei einer Konnektion nicht komplementärer Konnektorteile die Verabreichung einer falschen Behandlungsflüssigkeit zuverlässig vermieden wird.

Erfindungsgemäß ist somit vorgesehen, dass das Öffnungselement des einen Konnektorteils nur dann das Septum oder dergleichen, vorzugsweise das Schlitzseptum des anderen Konnektorteils durchbrechen bzw. durchdringen kann, wenn der Vorsprung und die Aufnahme an den Konnektorteilen komplementär sind, d.h. wenn die Konnektorteile zueinander passen. Ist dies nicht der Fall, kann das Öffnungselement das Septum nicht durchbrechen oder öffnen, sodass eine fehlerhafte Infusion bzw. Verabreichung einer Behandlungsflüssigkeit zuverlässig verhindert wird.

Bei dem Öffnungselement kann es sich vorzugsweise um einen Fluidkanal eines der Konnektorteile handeln.

Die vorliegende Erfindung betrifft des Weiteren einen Konnektor umfassend wenigstens ein erstes Konnektorteil und wenigstens ein zweites Konnektorteil, wobei die Konnektorteile miteinander fluiddicht verbindbar und über wenigstens ein Verschlusselement aneinander arretierbar sind, wobei eines der Konnektorteile wenigstens ein Dichtelement mit Öffnung, insbesondere einen Dichtring, und das andere der Konnektorteile wenigstens eine Anlagefläche für das Dichtelement aufweist, und wobei eines der Konnektorteile wenigstens einen Vorsprung und das andere der Konnektorteile wenigstens eine Aufnahme aufweist, wobei das Dichtelement und die Anlagefläche derart zueinander angeordnet sind, dass das eine fluiddichte Verbindung zwischen dem ersten und dem zweiten Konnektorteil nur dann besteht, wenn der Vorsprung in die Aufnahme eingeführt ist.

Ist die Verbindung der beiden Konnektorteile nicht korrekt ausgeführt, zeigt eine Leckage dies an, weil in diesem Zustand keine fluiddichte Verbindung zwischen den beiden Konnektorteilen vorliegt. Eine fluiddichte Verbindung, bei der keine Leckage nach außen hin auftritt, liegt in dieser Ausführungsform der Erfindung nur vor, wenn die Verbindung korrekt hergestellt ist, d.h. wenn der genannte Vorsprung in die Aufnahme eingeführt ist.

Anstelle des Dichtrings kann auch jedes andere geeignete Dichtelement verwendet, so dass der Begriff "Dichtring" nicht nur für einen Ringdichtung, sondern stellvertretend für umfängliche Dichtelemente (z.B. Dichtmanschette, Lochscheibe, eckige Ringe etc.) mit einer Öffnung zur Aufnahme eines Anschlussstutzens steht.

Die vorliegende Erfindung betrifft des Weiteren einen Konnektor umfassend wenigstens ein erstes Konnektorteil und wenigstens ein zweites Konnektorteil, wobei die Konnektorteile miteinander fluiddicht verbindbar und über wenigstens ein Verschlusselement aneinander arretierbar sind, wobei eines der Konnektorteile wenigstens einen Vorsprung und das andere der Konnektorteile wenigstens eine Aufnahme aufweist, wobei der Vorsprung bei komplementären Konnektorteilen in die Aufnahme einführbar ist.

Dabei ist erfindungsgemäß vorgesehen, dass der Vorsprung oder die Aufnahme an einem Zusatzteil, vorzugsweise an einem ringförmigen oder hülsenförmigen Zusatzteil angeordnet ist, das über einen Abschnitt des Konnektorteils und vorzugsweise über dessen Grundkörper geführt ist. In diesem Fall ist der Vorsprung und/oder die Aufnahme an einem separaten Teil angeordnet, das über den Grundkörper gesteckt wird. Dieser Aufbau ist insbesondere aus wirtschaftlichen Gesichtspunkten vorteilhaft, da nur ein einziger Grundkörpertyp hergestellt werden muss, der mit einer Vielzahl von Codiermitteln und Verschlussmitteln kombiniert werden kann.

Auch eine Kombination zweier oder aller der drei erfindungsgemäßen Gedanken gemäß der Ansprüche 1 bis 3 ist denkbar und von der Erfindung mit umfasst. So ist es denkbar, dass der Konnektor gemäß Anspruch 3 gemäß der Merkmale der Ansprüche 1 oder 2 oder gemäß der Merkmale des Anspruchs 1 und zusätzlich gemäß der Merkmale des Anspruchs 2 ausgebildet ist. Auch ist es denkbar, dass der Konnektor gemäß der Merkmale des Anspruchs 1 zusätzlich gemäß der Merkmale des Anspruchs 2 ausgebildet ist.

In einer weiteren Ausgestaltung der Erfindung ist vorgesehen, dass das Verschlusselement durch wenigstens einen Stift und durch wenigstens eine nutförmige Aufnahme für diesen Stift gebildet wird, wobei vorzugsweise vorgesehen ist, dass die Nut derart ausgebildet ist, dass sich diese über einen Teilbereich in einer axialen Richtung des Konnektorteils und über einen Teilbereich in Umfangsrichtung des Konnektorteils erstreckt, sodass zur Arretierung eine Schiebe- und eine Drehbewegung der beiden Konnektorteile relativ zueinander erforderlich ist. In diesem Fall erfolgt die Verbindung der beiden Konnektorteile somit vergleichbar mit einem Bajonett-Verschluss.

An der Nut können eine oder mehrere Rastelemente vorgesehen sein, die von dem Stift bei Erreichen der Arretierungsstellung beider Kontaktteile kontaktiert werden. Diese Rastelemente können als Sicherung der Arretierung dienen und darüber hinaus beim Überstreichen des Stiftes ein Geräusch verursachen, sodass für den Nutzer akustisch wahrnehmbar ist, dass die Arretierungsstellung erreicht ist. Alternativ oder zusätzlich ist ein haptisches Feedback bei Erreichen der Arretierungsstellung, d.h. wenn der Stift über das Rastelement schnappt, denkbar und von der Erfindung mit umfasst.

Vorzugsweise ist vorgesehen, dass der Stift bei Erreichen der Arretierungsstellung sichtbar ist, sodass auch eine optische Kontrolle der Arretierungsposition möglich ist.

In weiterer Ausgestaltung der Erfindung ist vorgesehen, dass das Zusatzteil oder eine sonstige farbliche Markierung, die sich auf dem ersten Konnektorteil befindet, farblich auf das zweite Konnektorteil abgestimmt ist. Entsprechendes kann auch für das zweite Konnektorteil gelten. So ist es über eine farbliche Ausgestaltung der beiden Konnektorteile oder von Zusatzelementen, wie Ringen etc. möglich, anzuzeigen, dass die beiden Konnektorteile komplementär sind, d.h. miteinander verbunden werden können.

Vorzugsweise ist vorgesehen, dass das Zusatzteil oder das sonstige Markierungselement derart angeordnet sind, dass diese auch im konnektierten Zustand der Konnektorteile sichtbar sind, sodass auch eine optische Kontrolle der komplementären Konnektorteile möglich ist.

In einer weiteren Ausgestaltung der Erfindung ist vorgesehen, dass das Zusatzteil eine Verpressfläche für das Dichtelement, wie beispielsweise die Ringdichtung oder das Septum aufweist und dass das Konnektorteil, auf dem das Zusatzteil angeordnet ist, eine Dichtfläche aufweist, an der das Dichtmittel, wie beispielsweise die Ringdichtung oder das Septum anliegt. In diesem Fall erfüllt das Zusatzteil nicht nur die Funktion, den Vorsprung bzw. die Aufnahme zu tragen, sondern zusätzlich auch noch die der Fixierung des Dichtmittels, insbesondere der Ringdichtung oder des Septums an dem Konnektorteil.

Der im Folgenden verwendete Begriff "Dichtelement" ist übergreifend zu verstehen. Er steht nicht nur für Dichtscheiben nach der Art eines Septums, sondern stellvertretend für alle beliebigen Dichtelemente, die dichtend aufmachbar sind oder bereits eine Öffnung zur Aufnahme einer Dichtfläche eines Stutzens besitzen.

In einer weiteren Ausgestaltung der Erfindung ist vorgesehen, dass das erste oder das zweite Konnektorteil einen Grundkörper aufweist, auf den das Zusatzteil aufgebracht ist, wobei der Grundkörper eine oder mehrere Rastnasen aufweist, die in wenigstens einem Durchbruch in dem Zusatzteil eingreifen, wobei die Verbindung zwischen Rastnasen und Durchbruch vorzugsweise derart ausgebildet ist, dass diese nicht manuell lösbar ist. Somit ist es beispielsweise denkbar, auf einen Grundkörper bzw. auf einen Abschnitt des Grundkörpers das Zusatzteil aufzustecken und z. B. durch eine Verrastung zu arretieren, wobei vorzugsweise vorgesehen ist, dass das Zusatzteil nach dem Herstellen der Rastverbindung nicht oder nur unter erheblichem Kraftaufwand von dem Grundkörper gelöst werden kann.

Weiterhin kann vorgesehen sein, dass der wenigstens eine Vorsprung als Erhebung ausgeführt ist, die sich in Längsrichtung und in Umfangsrichtung eines Konnektorteils erstreckt und/oder sich die Aufnahme in Längsrichtung und in Umfangsrichtung des anderen Konnektorteils erstreckt.

Vorzugsweise ist dabei vorgesehen, dass die Vorsprünge und die Aufnahme bei komplementären Konnektorteilen so dimensioniert sind, dass in die Aufnahme der wenigstens eine Vorsprung aufnehmbar ist und dass bei zueinander nicht komplementären Konnektorteilen dies nicht der Fall ist. So ist es beispielsweise denkbar, dass bei komplementären Konnektorteilen die Vorsprünge vollständig in die Aufnahme eingeschoben werden können und bei nicht komplementären Konnektorteilen dies nicht der Fall ist. Dies hat zur Folge, dass bei nicht komplementären Konnektorteilen ein Zusammenschieben der Konnektorteile wenn überhaupt nur soweit möglich ist, dass das Öffnungselement das Septum nicht öffnet, wie dies oben näher ausgeführt ist.

In einer weiteren Ausgestaltung der Erfindung ist vorgesehen, dass das Öffnungselement als vorzugsweise konisch zulaufender Stutzen ausgebildet ist, der im konnektierten Zustand der Konnektorteile einen Fluid durchströmten Bereich bildet. Dieser Stutzen ist gegenüber dem offenen Endbereich des Konnektorteils, an dem die Konnektierung mit dem anderen Konnektorteil vorgenommen wird als Berührschutz zurückversetzt.

Weiterhin kann vorgesehen sein, dass beide Konnektorteile Griffflächen aufweisen, die im arretierten Zustand der Konnektorteile in einer gemeinsamen Fläche liegen und die im nicht arretierten Zustand der Konnektorteile nicht in einer gemeinsamen Fläche liegen. So ist auch eine optische Kontrolle ohne Weiteres dahingehend möglich, ob die beiden Konnektorteile in der gewünschten Position arretiert wurden oder ob dies nicht der Fall ist. Auch in diesem Fall ist alternativ oder zusätzlich eine haptische Kontrolle bei Erreichen der gewünschten Position der Konnektorteile denkbar, was ebenfalls von der Erfindung mitumfasst ist.

Weitere Einzelheiten und Vorteile der Erfindung werden anhand eines in den Zeichnungen dargestellten Ausführungsbeispiels näher erläutert. Es zeigen:
- Figur 1:: eine perspektivische Ansicht des Grundkörpers eines Konnektorteils,
- Figur 2:: eine perspektivische Ansicht eines Septums,
- Figur 3:: eine perspektivische Darstellung des Zusatzteils,
- Figur 4:: unterschiedliche Ansichten des zweiten Konnektorteils,
- Figur 5:: unterschiedliche Ansichten einer Schutzkappe als Einzelteil und im aufgesetzten Zustand,
- Figur 6:: eine perspektivische Ansicht des zweiten Konnektorteils mit FarbCodierring,
- Figur 7:: unterschiedliche Ansichten des Konnektors im arretierten und im nicht arretierten Zustand,
- Figur 8:: eine Schnittdarstellung durch den Konnektor im arretierten Zustand,
- Figur 9:: perspektivische Darstellungen zweier Konnektorteile, die nicht komplementär sind und
- Figur 10:: eine Darstellung von Konnektorteilen, die zueinander komplementär sind.

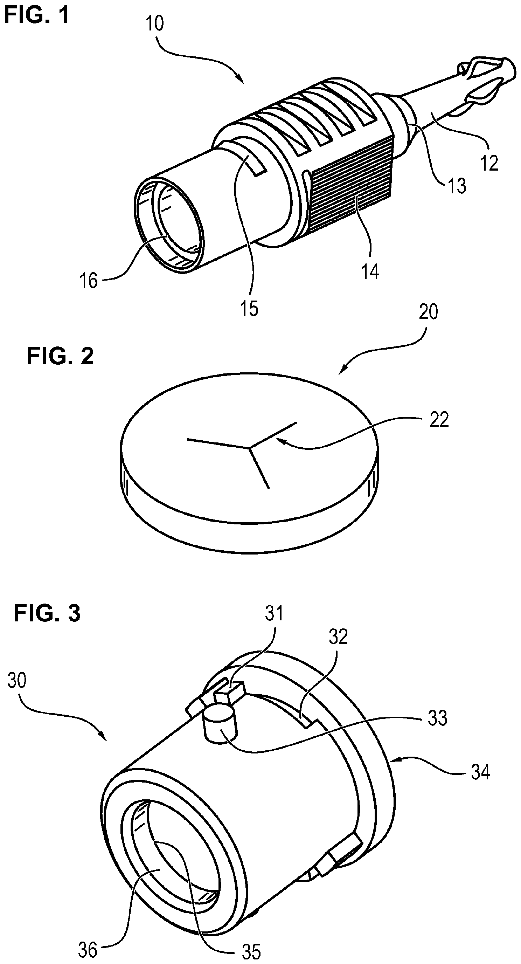

Figur 1 zeigt den Grundkörper 10, der zusammen mit dem in Figur 3 dargestellten Zusatzteil (im Folgenden: Codierring) das erste Konnektorteil bildet.

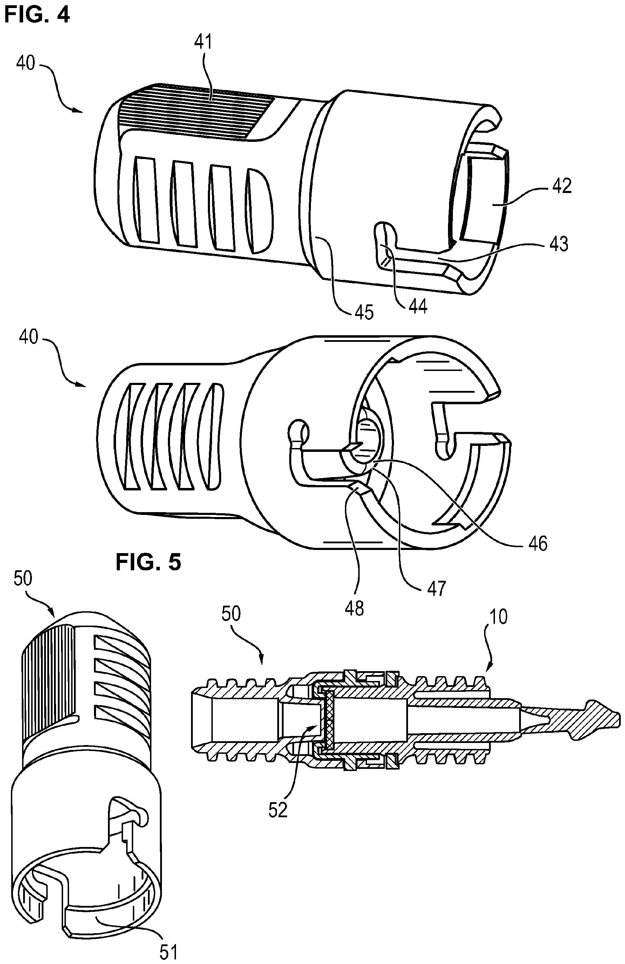

Das zweite Konnektorteil wird durch den in Figur 4 dargestellten Schlauchkonnektor 40 gebildet.

Der in Figur 1 dargestellte Grundkörper bleibt bei allen Varianten des Konnektors gleich.

Dies bringt den Vorteil mit sich, dass ein eventueller Kontakt mit einer medizinischen Lösung oder einem Pharmazeutikum mit immer nur einer Komponente besteht.

Da die FarbCodierung über die anderen Konnektorbestandteile, nämlich über den Codierring 30 und den Schlauchkonnektor 40 vorgenommen wird, kann bei dem Grundkörper 10 gemäß Figur 1 auf jeglichen Eintrag durch eluierbare oder kontaktaktive Farbgranulate verzichtet werden. Auf diese Weise werden Wechselwirkungen zwischen etwaigen Farbgranulaten und medizinischen Lösungen auf ein Minimum reduziert, da es keinerlei Materialvariationen im direkten Kontakt mit den Arzneimitteln bzw. Lösungen gibt.

In Figur 1 zeigt das Bezugszeichen 12 einen Abbrechkonus und das Bezugszeichen 13 einen Schlauchsitz (Lösungsseite) zum Aufstecken eines Schlauches. Diese beiden Teile bilden die lösungsberührende Seite.

Das Bezugszeichen 14 kennzeichnet eine Grifffläche des Grundkörpers bzw. des ersten Konnektorelementes, die abgeflacht ist und die durch ihre Haptik und Optik intuitiv die richtige Konnektionsposition vorgibt.

Mit dem Bezugszeichen 15 ist eine vorzugsweise umlaufende Rastnase für den in Figur 3 dargestellten Codierring gezeigt. Optional ist auch eine Schweißverbindung möglich.

Das Bezugszeichen 16 kennzeichnet die Dichtfläche für das in Figur 2 exemplarisch dargestellte Dichtelement.

Das Dichtelement gemäß Figur 2 mit dem Bezugszeichen 20 kann beispielsweise eine Dichtscheibe aus Silikon sein, die einen geschlitzten Durchbruch 22 aufweist. Optionale Dichtelemente sind ein O-Ring, eine Dichtscheibe oder auch ein anderweitig elastisches Element.

Grundsätzlich ist auch eine andere Gestaltung des Dichtelementes bzw. durch die Einbringung eines weiteren Dichtelementes auch eine Doppeldichtung realisierbar, was allerdings mit einem erhöhten Aufwand und erhöhten Kosten einhergeht.

Das Bezugszeichen 30 in Figur 3 kennzeichnet den Codierring, der auf den in Figur 1 links dargestellten Abschnitt des Grundkörpers aufgesteckt wird.

Der Codierring erfüllt die Aufgaben der mechanischen Codierung mit dem in Figur 4 dargestellten Schlauchkonnektor, eine farbliche Codierung sowie das Verpressen und Halten des Dichtelementes vorzugsweise ohne Schweißen oder Kleben.

Eine Verdrehsicherung des Codierrings 30 ist durch die Geometrie der Rastnasen 15 bzw. der Schweißnasen und der Durchbrüche 32 des Codierrings gegeben. Durch die entsprechende Formgebung der Rastnasen am Grundkörper wird der Codierring vorzugsweise unlösbar auf dem Grundkörper 10 aufgeschnappt bzw. dort verschweißt oder anderweitig fixiert.

In Figur 3 zeigt das Bezugszeichen 31 Codierstifte, d.h. die Vorsprünge im Sinne der Erfindung, deren Positionen von Konnektor zu Konnektor variieren können.

Diese Codierstifte erstrecken sich wie aus Figur 3 ersichtlich zum einen in Längsrichtung des Codierrings 30 und zum anderen in Umfangsrichtung.

Das Bezugszeichen 32 kennzeichnet den Durchbruch für die Rastnasen 15 des Grundkörpers 10 und dienen gleichzeitig als Verdrehsicherung des Codierrings 30.

Mit dem Bezugszeichen 33 ist ein Stift für den Bajonett-Verschluss gekennzeichnet, der mit einer Nut des Schlauchkonnektors 40 gemäß Figur 4 zusammenwirkt und so das Verschlusselement bildet.

Mit dem Bezugszeichen 34 ist ein auch im konnektierten Zustand sichtbarer Ring des Codierrings 30 gekennzeichnet, der zur Farbcodierung dient. Vorzugsweise ist der gesamte Codierring 30 eingefärbt.

Das Bezugszeichen 35 kennzeichnet schließlich eine Verpressfläche, die innenliegend ist und die zum Verpressen des Dichtelementes dient, das seinerseits an der Dichtfläche 16 des Grundkörpers 10 gemäß Figur 1 anliegt. Das Dichtelement wird somit zwischen dem Grundkörper und dem Codierring verpresst.

Mit dem Bezugszeichen 36 ist ein Rücksprung gekennzeichnet, der als Berührschutz des Dichtelementes dient.

Durch die Einfärbung und durch die mechanische Codierung ist die Verbindung eindeutig und unverwechselbar.

Wie oben ausgeführt, zeigt Figur 4 den Schlauchkonnektor, der das Gegenstück zu dem Grundkörper 10 und zu dem Codierring 30 bildet.

Vorzugsweise passt nur der jeweils richtige Schlauchkonnektor auf den entsprechend passenden Codierring 30. Die Rückmeldung einer erfolgten und sicheren Konnektierung erfolgt sowohl optisch als auch akustisch und haptisch.

Wie dies aus Figur 4 hervorgeht, weist der Codierring abgeflachte Griffflächen 41 auf, die intuitiv die richtige Konnektionsposition vorgeben. Im konnektierten Zustand liegen diese Griffflächen in einer Ebene mit den entsprechenden Flächen 14 des Grundkörpers 10.

Das Bezugszeichen 42 kennzeichnet die Aufnahme im Sinne der vorliegenden Erfindung, die als innen liegende Aussparung ausgebildet ist, die sich in Umfangsrichtung erstreckt.

Diese Aussparung dient für die Aufnahme der Codierstifte 31 des Codierrings 30. Deren Position kann von Konnektor zu Konnektor variieren.

Mit dem Bezugszeichen 43 ist eine Öffnung für den Stift 33 des Bajonett-Verschlusses am Codierring 30 gekennzeichnet. Wie dies aus Figur 4 hervorgeht, erstreckt sich die Nut zur Aufnahme des Stiftes 33 zum einen in Längsrichtung des Schlauchkonnektors und zum anderen in radialer Richtung.

Mit dem Bezugszeichen 44 sind Rastelemente gekennzeichnet, die zur Sicherung der Konnektion gegen ein Selbstlösen der Verbindung und zur optischen und haptischen Rückmeldung dienen. Somit ist für einen Nutzer ohne Weiteres feststellbar, dass ein Einrasten und damit eine hinreichende Arretierung erfolgt ist.

Die Farbcodierung, die eine eindeutige Zuordnung zwischen dem Schlauchkonnektor und dem Grundkörper bzw. dem Codierring 30 liefert kann beispielsweise über einen komplett eingefärbten Schlauchkonnektor 40 erfolgen, der die gleiche Farbe aufweisen kann wie der Codierring 30 oder auch über einen an einer Position aufgeschnappten oder eingefärbten Ring. Wesentlich ist eine farbliche Zuordnung zwischen dem Schlauchkonnektor einerseits und dem ersten Konnektorteil, d.h. dem Grundkörper bzw. dem Codierring 30 andererseits.

Mit dem Bezugszeichen 46 ist ein Konus, d.h. das Öffnungselement im Sinne der vorliegenden Erfindung gekennzeichnet. Dieser Konus dient zur Abdichtung der Verbindung mit Hilfe eines elastischen Dichtelementes gemäß Figur 2 am Grundkörper.

Mit dem Bezugszeichen 47 ist ein Rücksprung des Konusses als Berührschutz vorgesehen und mit dem Bezugszeichen 48 eine Abschrägung zur leichteren Feinpositionierung.

Das Bezugszeichen 50 in Figur 5 kennzeichnet eine Schutzkappe, die auf den mit dem Codierring 30 versehenen Grundkörper 10 aufgesteckt werden kann. Wie dies aus Figur 5 hervorgeht, weist diese Schutzkappe 50 eine umlaufende Codieraussparung 51, sodass die Schutzkappe 50 auf jeden beliebigen Codierring 30 aufgesetzt werden kann und somit universell ist. Mit diesem Prinzip wäre auch der Einsatz eines Universaladapters realisierbar, der z.B. die Möglichkeit hat, auf allen Konnektoren einen Standard-Luer-Anschluss bereitzustellen.

Figur 5, rechte Darstellung zeigt eine Schnittdarstellung und verdeutlicht, dass in der Position 52 eine Abstützung der Dichtscheibe gemäß Figur 2 erfolgt, um bei Nichtbenutzung die Dichtscheibe geschlossen und damit dicht zu halten.

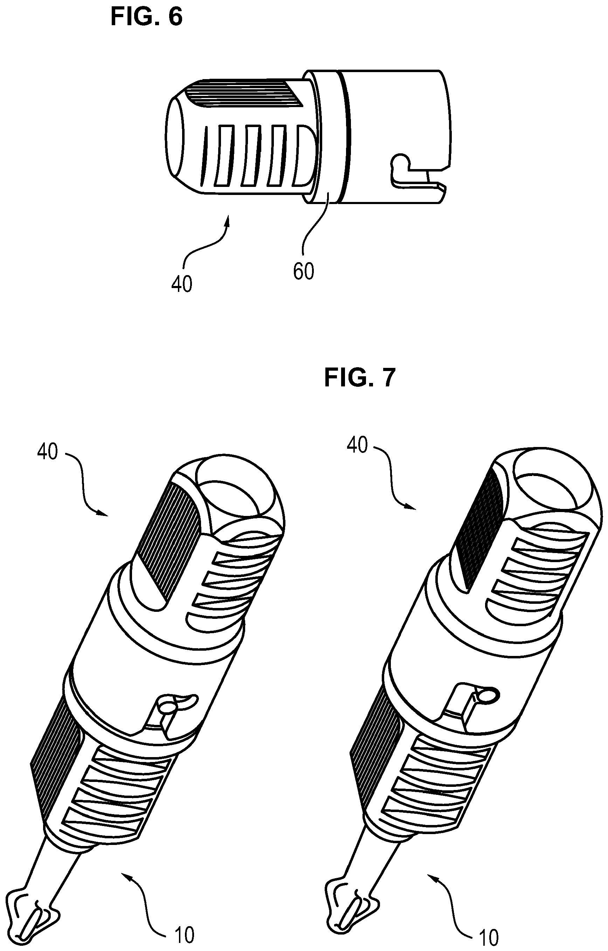

In Figur 6 ist der Schlauchkonnektor, d.h. das zweite Konnektorteil gemäß der Erfindung gekennzeichnet. Wie dies aus Figur 6 hervorgeht, ist ein aufgeschnappter Farbcodierring 60 vorgesehen, der eine andere Farbe aufweisen kann, als der Schlauchkonnektor selbst. Falls eine einfarbige Codierung bzw. eine Einfärbung des Schlauchkonnektors selbst nicht ausreichen sollte, kann durch einen oder mehrere solcher Farbcodierringe 60 die Farbpalette beliebig erweitert werden. Dies gilt selbstverständlich nicht nur für die Seite des Schlauchkonnektors, sondern auch für die Seite des Grundkörpers bzw. des Codierrings.

Durch diese Farbcodierung in Form des Ringes wäre es auch möglich, in den lösungsberührenden Bereichen des gesamten Konnektors komplett auf eingefärbte Kunststoffe zu verzichten.

Aus Figur 7 geht das Prinzip der Konnektion der beiden Konnektorteile hervor.

Gemäß Figur 7, linke Darstellung ist die Verrastung bzw. Arretierung offen und in Figur 7, rechte Darstellung geschlossen.

Wie dies aus Figur 7, rechte Darstellung hervorgeht, ist in der arretierten bzw. geschlossenen Position der Griffbereich des ersten Konnektorteils und der Griffbereich des zweiten Konnektorteils in einer Ebene. Der Griffbereich des Konnektors ist somit so gestaltet, dass sich beim Anfassen ergonomisch eine Vorzugslage in den Fingern ergibt, d.h. eine Vorpositionierung gemäß Figur 7, linke Darstellung. Durch die Drehbewegung wird dann die in Figur 7, rechte Darstellung erreicht.

Durch die Vorpositionierung wird die Findung der Rastelemente deutlich erleichtert.

Wie dies weiter aus Figur 7 hervorgeht, weist sowohl das erste als auch das zweite Konnektorteil einen bauchigen Bereich auf, dessen Radius zur Konnektorachse größer ist als der Abstand der Griffbereiche zur Konnektorachse und der rund ausgeführt ist. Des Weiteren weisen beide Konnektorteile einen oder mehrere nahezu flache bzw. ebene Bereiche auf, der den Griffbereich bildet und gegenüber dem Außenumfang des gekrümmten Bereiches zurückversetzt ist. Die Riffelung in den Griffbereichen gibt auch bei feuchtem Konnektor Halt. Einführschrägen an den Kappen erleichtern die Feinpositionierung.

Wie dies aus Figur 7, rechte Darstellung hervorgeht, ist in der arretierten Position der Stift 33 des Codierrings 30 im Endbereich der Nut des Schlauchkonnektors 40 aufgenommen und wird dort durch die genannten Rastelemente fixiert. Auf diese Weise ist auch eine optische Kontrolle der vollständigen Arretierung möglich.

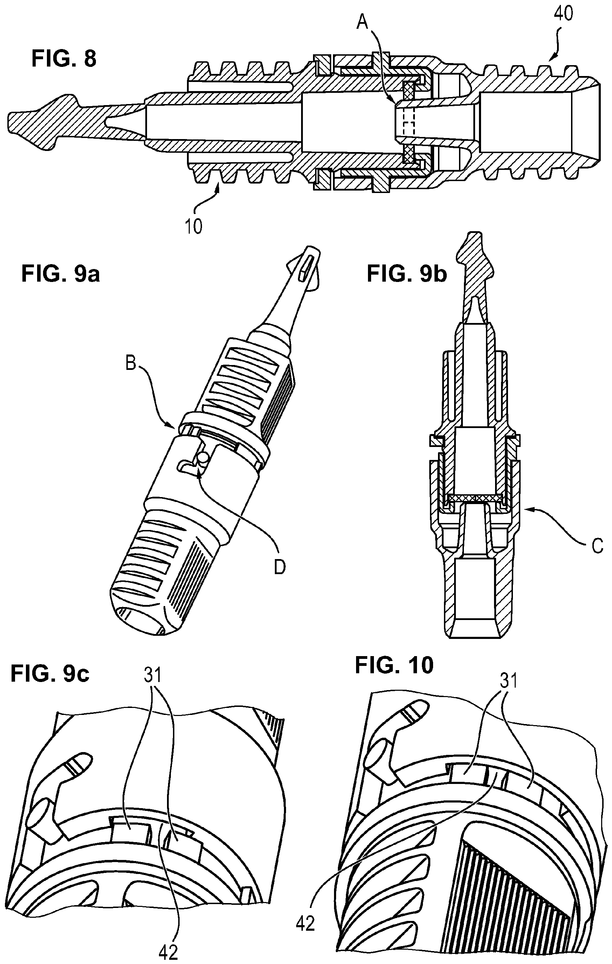

Figur 8 verdeutlicht in einer Schnittdarstellung, dass an der Stelle A im konnektierten und arretierten Zustand der Konus 46 die Dichtscheibe 20 durchdringt und diese öffnet, so dass eine Durchströmung des Konnektors möglich ist.

Der Konnektor ist somit korrekt verbunden.

Figur 9 zeigt eine Situation, in der zwei nicht komplementäre, d.h. "falsche" Konnektionsteile miteinander verbunden werden sollen.

Wie dies aus Figur 9a hervorgeht, befindet sich in diesem Fall an der Stelle B ein Spalt und aufgrund der Tatsache, dass der Stift 33 noch nicht in dem in Umfangsrichtung verlaufenden Teil der Nut aufgenommen ist wird eine Drehung verhindert.

Wie dies aus Figur 9b hervorgeht, durchdringt der Konus in diesem Fall nicht die Dichtscheibe (vgl. Bezugszeichen C), sodass kein Durchfluss möglich ist.

Die Konnektion wird dadurch verhindert, dass gemäß Figur 9c die Codierstifte, d.h. die Vorsprünge breiter ausgeführt sind, als die Aufnahme am Codierring, sodass ein Einführen nicht möglich ist.

Demgegenüber zeigt Figur 10 ein Ausführungsbeispiel mit komplementären Konnektorteilen, bei denen die Codierstifte 31 in die Ausnehmung 42 hineinpassen, sodass der Spalt B überwunden werden kann und die beiden Konnektorteile vollständig zusammengeschoben und dann verdreht werden können.

Weitere bevorzugte Eigenschaften und Vorteile des erfindungsgemäßen Konnektors werden im Folgenden näher beschrieben:
Es besteht eine maximale mechanische Vertauschsicherheit, d.h. bei falschen Konnektorteilen ist keine Konnektion und kein Durchfluss möglich, da der Konus bzw. das Öffnungselement noch vor der Dichtscheibe bzw. dem Septum stehen bleibt.

Eine sichere Handhabung wird durch eine farbliche Codierung und durch die intuitive Handhabung (grundsätzlich bekannter Bajonett-Verschluss) mit Positionierhilfe durch Griffflächen möglich.

Es existiert vorzugsweise kein Rückfederelement, das direkt am Stift angreift wie bei einem bekannten Bajonett-Verschluss. Eine Rückfederung ergibt sich durch das Septum, das in diesem Sinne nicht als Rückfederelement verstanden werden soll.

Bei der Diskonnektion gibt es einen Tropfschutz und eine Durchflusssperre bei Einsatz einer Dichtscheibe.

Durch die zurückgesetzten Dichtflächen besteht ein Berührschutz.

Die Farbcodierungen können variabel sein und beispielsweise durch Ringe oder sonstige Markierungen gebildet werden.

Langzeitproduktberührend kann nur eine gleichbleibende Komponente ausgeführt sein, ohne dass diese Farb- oder Materialvariationen aufweist.

Eine versehentliche Diskonnektion wird verhindert, da Rastelemente und Dichtelement ein alleiniges Zurückdrehen verhindern. Eine maximale Sicherheit gegen Diskonnektion bei plötzlichen auftretenden Zugbelastungen ist durch das Prinzip des Bajonett-Verschlusses gewährleistet. Im Gegensatz zu einer Schraubbewegung ist vorzugsweise kein Umgreifen beim Schließen des Konnektors nötig. Ausreichend ist eine Verdrehung um wenige Grad.

Dies ist auch im Hinblick darauf vorteilhaft, dass der Schlauch entsprechend nur wenig verdrillt wird.

Es existiert eine definierte Endposition der Verriegelung, definierte Verriegelungskräfte und Verriegelungsposition in Bezug auf die Dichtelemente im Inneren des Konnektors.

Die Konnektion kann nicht zu leicht und auch nicht zu fest ausgeführt sein. Sie sollte derart ausgeführt sein, dass sie nicht selbstlösend ist und auch nicht zu fest, damit sie grundsätzlich unlösbar ist.

Die Rückmeldung eines geschlossenen Konnektors kann durch die Lage der Griffflächen und durch den sichtbaren Bajonett-Verschluss optisch rückgemeldet werden. Auch eine akustische und haptische Rückmeldung durch Verrasten des Verschlusses ist denkbar und vorteilhaft.

Menschen mit einer Sehschwäche können den Konnektor auch weitgehend "blind" bedienen, da es für die richtige Konnektion nicht unbedingt nötig ist, eine Farbcodierung einzusetzen. Diese Personen können sich beispielsweise an der Orientierung der Griffflächen oder auch an der akustischen bzw. haptischen Rückmeldung der vollständigen Arretierung orientieren.

Vorzugsweise sind der Grundkörper gemäß Figur 1 und die Universalschutzkappe gemäß Figur 5 bei allen Konnektoren gleich, somit ist bei sehr großen Stückzahlen eine Kostenreduktion möglich, ohne dass Einbußen auf die Sicherheit vorliegen.

Vorzugsweise ist lösungsberührend immer die gleiche Komponente.

Das Prinzip der Universalschutzkappe gemäß Figur 5 kann auch auf einen Universaladapter umfunktioniert werden, der auf alle Konnektoren passt.

Der Codierring gemäß Figur 3 und das Dichtelement gemäß Figur 2 werden vorzugsweise ohne kleben und schweißen, sondern nur durch unlösbare Verschnappelemente in ihrer Position fixiert. Gleichwohl bleiben Schweißen und Kleben eine optionale Möglichkeit der Verbindung.

Die Anzahl der möglichen Permutationen ist theoretisch durch die freie Anzahl und Positionierbarkeit der Codierstifte unbegrenzt und wird nur durch das vorhandene Platzangebot des Konnektors, das letztlich durch die Größe des Konnektors bestimmt wird, eingeschränkt.

Durch Einfügen eines z.B. dritten Codierstiftes sind auch übergeordnete Lösungen denkbar, die auf mehrere, aber nicht alle Untergruppen passen. So ist es beispielsweise denkbar, dass ein Konnektor A und ein Konnektor B untereinander zwar inkompatibel sind, dass ein "Hauptschlüssel C" sowohl auf den Konnektor A als auch auf den Konnektor B passt. Der "Generalschlüssel D" kann dann wiederum auf alle Gegenstücke passen.

Das Ausführungsbeispiel und eine bevorzugte Ausgestaltung der Erfindung betrifft eine bajonettähnliche Schließeinheit, ohne dass ein Rückfederelement nötig wäre sowie die durch die Codierstifte mechanische Codierung der Verbindung.

Die Funktionsweise des Konnektors ist selbsterklärend, intuitiv und verwechslungssicher, mit einer Mehrzahl von Rückmeldungen an den Anwender, ob die Verbindung korrekt geschlossen wurde oder nicht. Fehlbedienungen sind auch bei ungeschultem Fachpersonal weitgehend ausgeschlossen. Die Bedienbarkeit ist auch für Laien und Blinde intuitiv durchzuführen, auch für Patienten, die selbst konnektieren, z.B. in der Heimdialyse.

Wie oben ausgeführt ist die Anzahl der möglichen Codierungen nur vom Umfang des Konnektors beschränkt. Es ist möglich, einen Konnektor als Generalschlüssel zu verwenden, um mehrere Hauptschlüssel mit Untergruppen in dieser Codierungsart umzusetzen.

Eingangs ausgeführt wurde der Anwendungsbereich der Dialyse. Die vorliegende Erfindung ist jedoch nicht auf diesen Anwendungsbereich beschränkt. Auch ist nicht unbedingt eine Beschränkung auf eine medizinische Indikation vorgesehen. Überall dort, wo eine verwechslungs- und berührungssichere Verbindung von fluid- und/oder gasdurchflossener Leitung im Niederdruckbereich benötigt wird ist die erfindungsgemäße Konnektion vorstellbar. Dabei ist es ohne Bedeutung, ob das Prinzip mit einem einmal oder mit einem mehrmals verwendeten Konnektor angewandt wird.

Die Vorteile des Konnektor lassen sich je nach Ausführungsform wie folgt benennen:
- Maximale mechanische Vertauschbarkeit, d. h. bei falschen Konnektionspartnern ist keine Konnektion und kein Durchfluss möglich, da der Konus noch vor der Dichtscheibe stehen bleibt.
- Farbliche Codierung
- Intuitive Handhabung (Prinzip des Bajonettverschlusses ist jedem bekannt und auf den ersten Blick ersichtlich. Positionierhilfe erhält der Anwender durch vorgegebene Griffflächen).
- Kein konstruktiv eigenes Rückfederelement nötig (Bei Verwendung einer Dichtscheibe, tritt aber ein leichter Rückfedereffekt auf, der für das Handling des Konnektors durchaus von Vorteil sein kann.)
- Tropfschutz und Durchflusssperre bei Diskonnektion (bei Einsatz einer Dichtscheibe)
- Berührschutz durch zurückgesetzte Dichtflächen (gegenüber Standard-Luerverbindungen)
- Variable Farbcodierunge (optional)
- Langzeit-Produktberührend ist nur eine Komponente vorhanden, die für verschiedene Konnektoren mit verschiedenen Farb- und/oder Materialvariationen immer gleich ausgestaltet sein kann.
- Keine versehentliche Diskonnektion möglich, da Rastnasen und Dichtelement ein alleiniges Zurückdrehen verhindern. Maximale Sicherheit gegen Diskonnektion bei plötzlich auftretenden Zugbelastungen ist durch das Prinzip des Bajonettverschlusses gewährleistet.
- Im Gegensatz zu einer Schraubbewegung, ist kein Umgreifen beim Schließen des Konnektors nötig
- Der Schlauch wird nur um wenige Grad verdrillt.
- Definierte Endposition der Verriegelung, definierte Verriegelungskräfte und Verriegelungsposition in Bezug auf die Dichtelemente im Inneren des Konnektors
- Optische Rückmeldung eines geschlossenen Konnektors durch die ausgerichtete Lage der Griffflächen und durch den sichtbaren Bajonettverschluss.
- Akustische Rückmeldung, der Verschluss rastet hörbar ein.
- Haptische Rückmeldung, der Verschluss rastet spürbar ein.
- Menschen mit einer Sehschwäche können den Konnektor auch "blind" bedienen. Für die richtige Konnektion ist es nicht unbedingt nötig, die Farbcodierung des Konnektors zu erkennen.
- Grundkörper und Universalschutzkappe bzw. Zusatzteil sind bei allen Konnektoren gleich, damit ist bei sehr großen Stückzahlen eine Kostenreduktion möglich, ohne die Sicherheit zu gefährden.
- Aufbau einer universalen Schutzkappe oder eines universellen Adapters für alle Farb- und Kodiervariationen möglich.
- Der Codierring und das Dichtelement können ohne Kleben und Schweißen, sondern nur durch unlösbare Verschnappelemente an ihrer Position fixiert werden (Schweißen und Kleben bleibt aber eine optionale Möglichkeit der Verbindung).

## Patentansprüche

1. Konnektor umfassend wenigstens ein erstes Konnektorteil und wenigstens ein zweites Konnektorteil, wobei die Konnektorteile miteinander fluiddicht verbindbar und über wenigstens ein Verschlusselement aneinander arretierbar sind, wobei eines der Konnektorteile wenigstens ein im geschlossenen Zustand durchflusssperrendes, und öffenbares Dichtelement, insbesondere ein Septum oder eine Dichtscheibe, und das andere der Konnektorteile wenigstens ein Öffnungselement zum Öffnen des Dichtelementes aufweist und wobei eines der Konnektorteile wenigstens einen Vorsprung und das andere der Konnektorteile wenigstens eine Aufnahme aufweist, **dadurch gekennzeichnet, dass** das Öffnungselement und das Dichtelement derart zueinander angeordnet sind, dass das Öffnungselement das Dichtelement nur dann öffnet, wenn der Vorsprung in die Aufnahme eingeführt ist, wobei das Verschlusselement durch wenigstens einen Stift und durch wenigstens eine Nut für den Stift gebildet wird, und der wenigstens eine Vorsprung als Erhebung ausgebildet ist, welche sich in der Längsrichtung und der Umfangsrichtung eines Konnektorteils erstreckt, und / oder, dass sich die Aufnahme in der Längsrichtung und der Umfangsrichtung des anderen Konnektorteils erstreckt, wobei bei zueinander komplementären Konnektorteilen die Aufnahme derart dimensioniert ist, dass in dieser der wenigstens eine Vorsprung aufnehmbar ist und wobei bei zueinander nicht komplementären Konnektorteilen die Aufnahme derart dimensioniert ist, dass in dieser der wenigstens eine Vorsprung nicht aufnehmbar ist.

2. Konnektor nach Anspruch 1, **dadurch gekennzeichnet, dass** die Nut derart ausgebildet ist, dass sich diese über einen Teilbereich in einer axialen Richtung des Konnektorteils und über einen Teilbereich in Umfangsrichtung des Konnektorteils erstreckt, so dass zur Arretierung eine Schiebe- und eine anschließende Drehbewegung erforderlich ist.

3. Konnektor nach Anspruch 1 oder 2, **dadurch gekennzeichnet, dass** an der Nut ein oder mehrere Rastelemente vorgesehen sind, die von dem Stift bei Erreichen der Arretierungsstellung beider Konnektorteile kontaktiert werden.

4. Konnektor nach einem der vorhergehenden Ansprüche, **dadurch gekennzeichnet, dass** die Nut derart angeordnet ist, dass der Stift bei Erreichen der Arretierungsstellung sichtbar ist.

5. Konnektor nach einem der vorhergehenden Ansprüche, **dadurch gekennzeichnet, dass** das Öffnungselement als vorzugsweise konisch zulaufender Stutzen ausgebildet ist, der im konnektierten Zustand der Konnektorteile einen fluiddurchströmten Bereich bildet.

6. Konnektor nach einem der vorhergehenden Ansprüche, **dadurch gekennzeichnet, dass** das Öffnungselement gegenüber dem offenen Endbereich des Konnektorteils zurückversetzt ist.

7. Konnektor nach einem der vorhergehenden Ansprüche, **dadurch gekennzeichnet, dass** beide Konnektorteile Griffflächen aufweisen, die im arretierten Zustand der Konnektorteile in einer gemeinsamen Fläche liegen und die im nicht arretierten Zustand der Konnektorteile nicht in einer gemeinsamen Fläche liegen.

8. Erstes Konnektorteil, welches dazu ausgelegt ist, mit einem zweiten Konnektorteil zur Ausbildung eines Konnektors zusammenzuwirken, mit:
einem Grundkörper,
einem Stift eines Bajonett-Verschlusses,
einem Vorsprung, und
einem Dichtelement.

9. Erstes Konnektorteil nach Anspruch 8, weiterhin **gekennzeichnet durch** einen Griffabschnitt an dem Grundkörper, mittels dessen ein Benutzer das Konnektorteil ergreifen kann.

10. Erstes Konnektorteil nach Anspruch 8 oder 9, weiterhin **gekennzeichnet durch** einen Abbrechkonus und einen Schlauchssitz.

11. Erstes Konnektorteil nach einem der Ansprüche 8 bis 10, weiterhin **gekennzeichnet durch** eine Dichtfläche für das Dichtelement des Konnektorteils.

12. Erstes Konnektorteil nach einem der Ansprüche 8 bis 11, weiterhin **gekennzeichnet dadurch, dass** der Grundkörper eine oder mehrere Rastnasen aufweist, an welcher ein Zusatzteil, insbesondere ein Kodierring angebracht werden kann.

13. Erstes Konnektorteil nach einem der Ansprüche 8 bis 12, weiterhin **gekennzeichnet durch** einen Kodierring, welcher den Vorsprung als zumindest einen Kodierstift aufweist, welcher sich in Längsrichtung und in Umfangsrichtung des Kodierrings erstreckt, wobei der mindestens eine Stift für einen Bajonett-Verschluss an dem Kodierring angeordnet ist und dazu ausgelegt ist, mit einer auf einem zweiten Konnektorteil angeordneten Nut zusammenzuwirken.

14. Zweites Konnektorteil, welches dazu ausgelegt ist, mit einem ersten Konnektorteil zur Ausbildung eines Konnektors zusammenzuwirken, mit:
einem Grundkörper,
einer Nut eines Bajonett-Verschlusses,
einer Aufnahme, und
einem Öffnungselement.

15. Zweites Konnektorteil nach Anspruch 14, **dadurch gekennzeichnet, dass** die Aufnahme dazu ausgelegt ist, einen Vorsprung, insbesondere einen Kodierstift eines ersten Konnektorteils, aufzunehmen.

16. Zweites Konnektorteil nach Anspruch 14 oder 15, **dadurch gekennzeichnet, dass** die Nut eines Bajonett-Verschlusses dazu ausgelegt ist, den Stift eines Bajonett-Verschlusses aufzunehmen, wobei sich die Nut eines Bajonett-Verschlusses in einem ersten Abschnitt in der Längsrichtung des Grundkörpers erstreckt und sich in einem zweiten Abschnitt in Umfangsrichtung des Grundkörpers erstreckt.

17. Zweites Konnektorteil nach einem der Ansprüche 14 bis 16, **dadurch gekennzeichnet, dass** das Öffnungselement als Stutzen ausgebildet ist, welcher dazu ausgelegt ist, das Dichtelement eines ersten Konnektorteils zu durchdringen, wenn das erste Konnektorteil und das zweite Konnektorteil miteinander verbunden werden, um einen Konnektor auszubilden.

18. Zweites Konnektorteil nach einem der Ansprüche 14 bis 17, weiterhin mit Rastelementen, welche dazu ausgelegt sind, das erste Konnektorteil an dem zweiten Konnektorteil zu sichern.
